Europäisches Patentamt

European Patent Office

Office européen des brevets

(11) Veröffentlichungsnummer: **0 288 749**

**A2**

(12) **EUROPÄISCHE PATENTANMELDUNG**

(21) Anmeldenummer: 88104765.8

(51) Int. Cl.4: **A61F 13/02**

(22) Anmeldetag: 24.03.88

(30) Priorität: 30.04.87 DE 8706274 U

(43) Veröffentlichungstag der Anmeldung:
02.11.88 Patentblatt 88/44

(84) Benannte Vertragsstaaten:
AT BE CH DE ES FR GB GR IT LI LU NL SE

(71) Anmelder: LOHMANN GmbH & CO KG
Irlicher Strasse 55
D-5450 Neuwied 12(DE)

(72) Erfinder: List, Harald, Dr. med.
Am Schlosspark 60
D-5450 Neuwied 1(DE)

(74) Vertreter: Neidl-Stippler, Cornelia, Dr.
Rauchstrasse 2
D-8000 München 80(DE)

(54) **Haftkompresse.**

(57) Die Erfindung betrifft eine medizinische Kompresse mit einer Applikationsseite und einer Rückseite, einem flexiblen Saugteil und einer Befestigungseinrichtung, wobei der Saugteil (1) auf der Applikationsseite eine die Haftung der Kompresse sicherstellende haftklebende Befestigungseinrichtung (2) sowie ggf. eine obere Abdeckung auf der Rückseite trägt.

## Figur 1

Xerox Copy Centre

EP 0 288 749 A2

## Haftkompresse

Die Erfindung betrifft eine medizinische Kompresse mit einer Applikationsseite und einer Rückseite, flexiblem Saugteil und einer Befestigungseinrichtung,

Kompressen werden schon seit langem vor allem in der Wundbehandlung eingesetzt. Ihre Fixierung am Applikationsort wird dabei auf verschiedene Weise bewerkstelligt, wobei zwischen dem eigentlichen Saugkörper und der Fixierungvorrichtung unterschieden werden kann. So kann der Saugkörper, häufig Mull, Vliesmaterial od. dgl. nach Auflegen auf den Applikationsort, wie eine Wunde, durch nichtklebende Vorrichtungen wie Z.B. darübergewickelte Binden oder Schlauchverbände, fixiert werden oder es kommen haftklebende Vorrichtungen, wie Z.B. Heftpflaster oder Breitfixierpflaster, die über der körperabgewandten Seite des Saugkörpers und angrenzende Hautbereiche geklebt werden, zum Einsatz. Bei Breitfixierpflastern, die auch folienartig ausgebildet sein können, deckt das Pflaster den Saugkörper völlig ab und die überstehenden haftklebenden Randbereiche sorgen für die Fixierung. Eine ähnliche Konstruktion liegt bei den einteiligen sog. Wundschnellverbänden vor, bei denen schon vor Gebrauch der Saugkörper auf die Fixiereinrichtung mit der Rückseite aufgeklebt ist.

Diese schon bekannten Lösungen des Problems der Befestigung von Saugkompressen weisen jedoch Nachteile auf. So sind die mehrteiligen Vorrichtungen aufwendig, unbequem, teuer und erfordern einen hohen Zeitbedarf bei der Applikation. Des weiteren müssen an unebenen und insbesondere konkaven sowie der Körperbewegung stark ausgesetzten Applikationsorten meist zusätzliche Maßnahmen für eine dauerhafte Fixierung, sowie einen vollflächigen Kontakt ergrif fen werden, wie Fixierung mit Binden und Pflastern. In anderen Fällen müssen je nach Größe der Wunde verschiedene Formate bereitgestellt werden, was Probleme in der Lagerhaltung mit sich bringt.

Demgegenüber ist es Aufgabe der Erfindung, eine medizinische Kompresse mit einem flexiblen Saugteil mit einer Befestigungseinrichtung vorzuschlagen, die eine bessere vollflächige Befestigung bei besserer Handhabung der Kompresse ermöglicht.

Die Aufgabe wird erfindungsgemäß durch eine gattungsgemäße Kompresse gelöst, bei der der Saugteil auf der Applikationsseite eine haftklebende Befestigungseinrichtung sowie ggf. eine obere Abdeckung auf der Rückseite trägt.

Dabei kann die haftklebende Befestigungseinrichtung eine ununterbrochene, sekretdurchlässige Haftklebeschicht, eine durch nichtklebende Bereiche unterbrochene Haftklebeschicht oder voneinander isolierte Haftklebebereiche, sein, wobei es vorteilhaft sein kann, wenn die Applikationsseite eine vor Applikation der Kompresse entfernbare Abdeckschicht trägt.

Klebepunkte stellen nur eine mögliche Ausführung der Befestigung dar. Alle anderen möglichen geometrischen und verteilungsmäßigen Ausgestaltungen von isolierten Haftklebebereichen werden ebenfalls von der Erfindung umfaßt. Weiterhin ist es möglich, die Haftklebefläche durch nichtklebende Bereiche zu unterbrechen, wie z.B. eingitterförmiger Haftkleberauftrag, wobei widerum gilt , daß alle möglichen geometrischen und verteilungsmäßigen Anordnungen innerhalb der Erfindung liegen. Schließlich kann die Haftklebeschicht auch durchgehend ausgestaltet sein.

Hierbei sollte ein Haftklebertyp gewählt werden, der im Wundbereich durch das Sekret desintegriert und damit für das Sektret durchlässig wird. Allgemein sind alle hypoallergenen Haftkleber geeignet. Dazu gehören beispielsweise soche auf Basis von Poyacrylat, Polyurethan, Silikon, Polyisobutylen und weiterhin Polyacrylat, Polyurethan, Silikon, Polyisobutylen und weiterhin Homo-, Co-oder Blockpolymerisate anderer geeigneter olefinisch ungesättigter Monomerer.

Die Kompresse kann zusätzlich auch einen umlaufenden Haftkleberand aufweisen.

Zur Verhinderung eines unkontrollierten Austretens von Flüssigkeit kann das Saugteil seitlich gegen Flüssigkeitsaustritt versiegelt sein.

Das Saugteil besteht üblicherweise im wesentlichen aus einem saugfähiges Material, wie Gewebe, Gewirke, Gelege, Gestricke, Geraschel oder Vliesstoff mit natürlichen und/oder synthetischen Fasern oder Fäden; einem offenzelliger Schaumstoff, wie Polyurethanschaum, Polyesterschaum oder Polyethylenschaum oder auch Schichtanordnungen derselben, wobei die einzelnen Schichten ganzflächig miteinander verklebt, vernadelt, vernäht oder in sonstiger Weise gegen Verrutschen und zur Sicherung des Flüssigkeitstransports miteinander verbunden sind. In jedem Falle muß die Wasserdampfdurchlässigkeit der Schichten gewährleistet sein.

Die erfindungsgemäße Kompresse zeichnet sich durch eine einfache und schnelle Handhabung aus, ist kostensparend und patientenfreundlich und garantiert selbst an unebenen, insbesondere an konkaven Applikationsorten eine rutsch sichere Fixierung und kann auf beliebige Größen zugeschnitten werden, insbesondere,wenn die Haftklebebereiche eine vollflächige Schicht oder auch regelmäßig auf der Applikationsseite verteilte Klebebereiche sind.

Sie kann in üblichen Verpackungen, bspw. steril, in den Handel gebracht werden.

Nachfolgend wird die Erfindung zur besseren Eriäuterung anhand der begleitenden Zeichnung, die eine bevorzugte Ausführungsform zeigt, näher erläutert. Es zeigen:

Fig. 1 den Aufbau einer erfindungsgemäßen Kompresse und

Fig. 2 eine Aufsicht auf die mit Hafkleber belegte Seite der Kompresse nach Fig.1 nach Abziehen der Abdeckschicht.

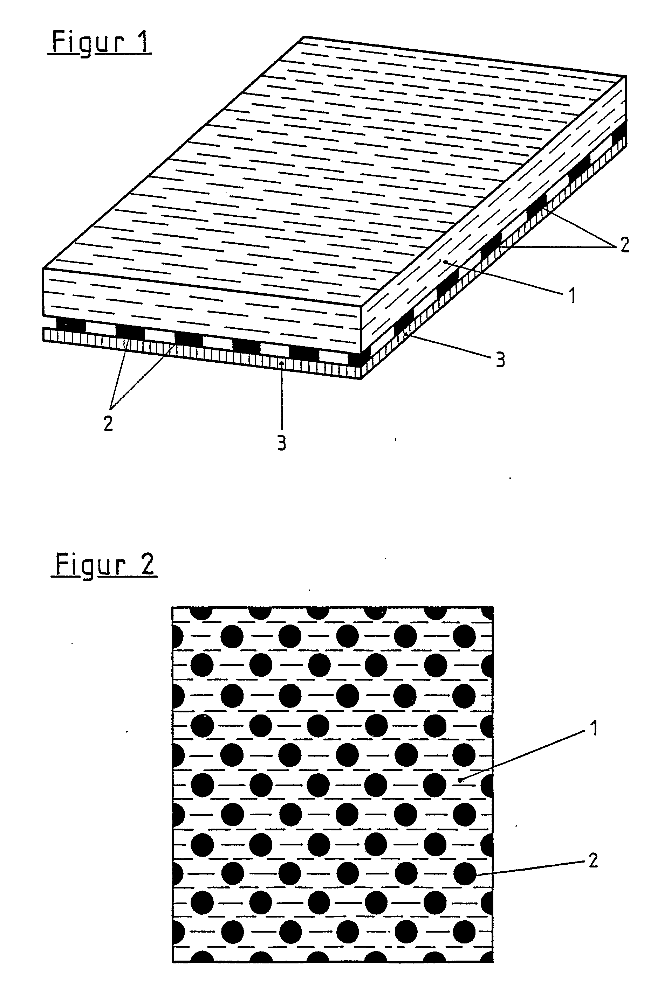

Die in Fig. 1 dargestellte Kompresse weist einen schichtförmigen, flexiblen Saugteil 1 auf, der hier aus einem textilen Flächenmaterial besteht. Der Saugteil 1 ist auf der Applikationsseite mit regelmäßig verteilten, voneinander getrennten Klebebereichen, die hier als Punkte ausgebildet sind, versehen, so daß eine Haftung des Saugteils am Applikationsort sichergestellt ist.

Selbstverständlich ist jede andere geometrische Form von Klebebereichen auswählbar und die Erfindung soll nicht nur auf runde Klebebereiche beschränkt sein.

Die haftklebende Befestigungseinrichtung der Kompresse ist mit einer Abdeckschicht 3 vor Gebrauch geschützt. Die dazu geeigneten Abdeckmaterialien, die zur Haftkleberseite hin abhäsiv ausgerüstet sein müssen, sind dem Fachmann geläu fig , wobei sich besonders Papiere und Polymer-sowie Metallfolien bewährt haben.

In Fig. 2 ist die Abdeckschicht 3 der Kompresse in Fig. 1 abgezogen, so daß der Blick auf die haftklebende Befestigungseinrichung 2 freigegeben wird. Auf dem Saugteil 1 sind die Haftklebepunkte 2 zu erkennen, die so auf der Fläche verteilt sind, daß ein sicherer Sitz der Kompresse am Applikationsort gewährleistet ist. Die kleberfreien Bereiche erlauben einen ungehinderten Zutritt des Wundsekrets zum Saugteil.

Nachfolgend wird noch eine bevorzugte Herstellung einer erfindungsgemäßen Kompresse beschrieben.

Beispiel

Eine wäßrige Polyacrylat-Dispersion mit einer Viskosität von 8 bis 9 mPas wird mittels eines Siebdruck-Auftragswerkes auf ein silikonisiertes Trennpapier (Abdeckung) derart aufgetragen, daß nach dem Trocknen diskrete Polyacrlyatkleberpunkte mit einem Flächengewicht von 20 -500 $g/m^2$ resultieren. Danach wird ein Nadelvlies von 120 $g/m^2$ zukaschiert, die Bahnware zu Rollen oder Formaten konfektioniert und in Primärpackmittel eingesiegelt. Gegebenenfalls schließt sich ein Sterilisationsvorgang an.

## Ansprüche

1. Medizinische Kompresse mit einer Applikationsseite und einer Rückseite, einem flexiblen Saugteil und einer Befestigungseinrichtung, dadurch **gekennzeichnet,** daß der Saugteil (1) auf der Applikationsseite eine die Haftung der Kompresse sicherstellende haftklebende Befestigungseinrichtung (2) sowie ggf. eine obere Abdeckung auf der Rückseite trägt.

2. Kompresse nach Anspruch 1, dadurch **gekennzeichnet,** daß die haftklebende Befestigungseinrichtung (2) eine ununterbrochene, sekretdurchlässige Haftklebeschicht ist.

3. Kompresse nach Anspruch 1, dadurch **gekennzeichnet,** daß die haftklebende Befestigungseinrichtung (2) eine durch nichtklebende Bereiche unterbrochene Haftklebeschicht ist.

4. Kompresse nach Anspruch 1, dadurch **gekennzeichnet,** daß die haftklebende Befestigungseinrichtung (2) voneinander isolierte Haftklebebereiche sind.

5. Kompresse nach einem der Ansprüche 1 bis 4, dadurch **gekennzeichnet,** daß die Befestigungseinrichtung (2) eine vor Applikation der Kompresse entfernbare Abdeckschicht (3) auf der Applikationsseite trägt.

6. Kompresse nach einem der vorangehenden Ansprüche, dadurch **gekennzeichnet,** daß der Saugteil (l)seitlich gegen Flüssigkeitsaustritt versiegelt ist.

7. Kompresse nach einem der vorhergehenden Ansprüche, dadurch **gekennzeichnet,** daß der Saugteil (1) im wesentli chen ein Gewebe, Gewirke, Gestricke, Geraschel oder ein Vliesstoff mit natürlichen und/oder synthetischen Fasern oder Fäden oder ein offenzelliger Schaumstoff, wie Polyurethanschaum, Polyesterschaum oder Polyethylenschaum,

8. Kompresse nach einem der vorangehenden Ansprüche, dadurch **gekennzeichnet,** daß der Haftkleber ein Polyacrylatkleber, ein Polyurethankleber, Silikonkleber oder Polyisobutylenkleber oder andere Homo-, Co-oder Blockpolymerisate olefinisch ungesättigter Monomerer ist.

## Figur 1

## Figur 2